# EUROPEAN PATENT APPLICATION

(11) **EP 2 143 798 A1**
(43) Date of publication of application: **13.01.2010**
(21) Application number: 08160030.6
(22) Date of filing: 09.07.2008
(51) Int. Cl.: C12N 15/82, C12N 9/02

(54) **A plant cell comprising enzymatic activities for converting glyoxylate to glycerate**

(71) Applicant: Universität zu Köln, 50923 Köln (DE)
(72) Inventor: Flügge, Ulf-Ingo Prof. Dr., 50997 Köln (DE); Maurino, Verónica G. Dr., 50939 Köln (DE)
(74) Representative: Dick, Alexander

(57) **Abstract**

The present invention is concerned with the improvement of agricobiological traits in plants. More specifically, it relates to a plant cell comprising enzymatic activities for the conversion of glyoxylate to glycerate. Preferably, said plant cell comprises a first polypeptide having tartronate semialdehyde synthase activity, and a second polypeptide having tartronate semialdehyde reductase activity. Also encompassed is a plant comprising said plant cells as well as seeds obtainable from the said plants. The present invention, furthermore, relates to a method for producing a transgenic plant or a plant cell having an increased salt tolerance and/or cold tolerance. The present invention contemplates polynucleotides comprising a combination of nucleic acids encoding the aforementioned polypeptides as well as vectors comprising the polynucleotides and uses thereof. Moreover the present invention also relates to a method for detecting a plant compound being capable of conferring increased cold tolerance and/or increased salt tolerance to a plant and/or plant cell.

## Description

The present invention is concerned with the improvement of agricobiological traits in plants. More specifically, it relates to a plant cell comprising enzymatic activities for converting glyoxylate to glycerate. Preferably, said plant cell comprises a first polypeptide having tartronate semialdehyde synthase activity, and a second polypeptide having tartronate semialdehyde reductase activity. Also encompassed is a plant comprising said plant cells as well as seeds obtainable from the said plants. The present invention, furthermore, relates to a method for producing a transgenic plant or a plant cell having an increased salt tolerance and/or cold tolerance. The present invention contemplates polynucleotides comprising a combination of nucleic acids encoding the aforementioned polypeptides as well as vectors comprising the polynucleotides and uses thereof. Moreover the present invention also relates to a method for detecting a plant compound being capable of conferring increased cold tolerance and/or increased salt tolerance to a plant and/or plant cell.

Abiotic stresses are serious environmental limitations for the growth of crop plants, reducing the yield of major crop plants by more than 50%. Two of the most limiting abiotic stresses regarding the production of agricultural crops are low temperatures and salinity.

Salinity has a dual effect on plant growth: a) via an osmotic effect on plant water uptake, b) via specific ion toxicities. A decrease of the osmotic potential of the soil solution results in a reduced access of the plant to soil water. If the soil dries, the salt concentration increases, leading to a further decrease of osmotic potential. In order to maintain water uptake from saline soil, plants must osmotically adjust, e.g. by the uptake of salts, or by synthesizing organic solutes (Sheldon et al., The effect of salinity on plant available water, SuperSoil 2004). However, only a few plants can tolerate high salt concentrations in the soil. The most agricultural crop plants only show limited growth, if at all, at high salt concentrations.

The main causes for salinization of soil are excessive use of water and inadequate drainage. Salinity is becoming widespread in many regions, and it there is evidence that serious salinization may affect more than 50 % of all arable crop land worldwide by the year 2050 (see Wang et al., Planta, 2003, 218:1-14).

Moreover, approximately two thirds of the world's landmass is annually subjected to temperatures below the freezing point resulting in a major limitation of plant growth. Chilling and freezing temperatures can significantly damage crop plants and, therefore, often adversely affect the yield of said plants. Low temperature may impose stress on a plant in a two-fold manner: By the effects of low temperature alone, and by dehydration of the cells and tissues when cellular water freezes (see Beck et al. (2004), J. Biosci. 29(4):449-459).

Although there is technology available that allows to mediate some problems caused by salinity and low temperatures, e.g. through drainage, irrigation with high quality water, growing plants in greenhouse or under plastic foil, irrigation with substances that allow frost protection , these measures cause high costs.

Thus, breeding of plants that are stress tolerant has become a promising strategy to mediate some problems that are caused by low temperature and salinity. Plants being salt tolerant could be grown on land with high levels of salt. Plants being cold tolerant could be grown in regions with a colder climate.

However, attempts to improve cold and/or salt tolerance by using conventional breeding technologies only had limited success. Recently, there have been attempts to improve cold and/or salt tolerance by engineering transgenic crops (see, e.g. e.g. antisense wheat plants for HKT1 (high affinity K⁺ transporter) have increaced salt tolerance (Laurie et al., Plant J, 2002, 32: 139-149), the overexpression of the Arabidopsis transcription factors CB1, -2 and -3 in *Brassica napus* confers freezing and drought tolerance (Jaglo et al., Plant Physiol, 2001, 127: 910-917), the overexpression of the Arabidopsis vacuolar Na⁺/H⁺ antiporter (AtNHX1) in tomato confers salt tolerance (Zhang and Blumwald, Nature Biotech., 2001, 19: 765-768). For a complete list of transgenic approaches performed until present see Chinnusamy et al., Crop. Sci, 2005, 45: 437-448 and Zhang et al., Plant Physiol, 2004, 135: 615-621. However, cold and salt tolerance are not conferred by the product of a single gene, but has turned out as a syndrome comprising many different traits of cell biology, such as fluidity of the biomembranes, synthesis and accumulation of low, molecular weight, and high molecular weight, cryoprotectants, increase of the potential to cope with oxidative stress and other stresses (see, e.g, Beck et al. (2004), *loc. cit*.)

Thus, there is a need for increasing cold tolerance and/or salt tolerance in plants in order to improve their agricobiological value. However, at the same time the aforementioned drawbacks shall be avoided.

Accordingly, the technical problem underlying the present invention could be seen as the provision of means and methods for complying with the aforementioned needs. The technical problem is solved by the embodiments characterized in the claims and herein below.

Accordingly, the present invention relates to a plant cell comprising enzymatic activities for converting glyoxylate into glycerate.

The term "plant cells" as used herein refers to cells of higher plants, preferably to cells of monocots and dicots. Moreover the term encompasses cells from all types of plant tissue, i.e. from leaves, roots, stems, vascular tissue, stalks, calli, cotelydons, stalks, anthers, petioles, seeds or embryonic plant tissue. The host cell according to the present invention can be obtained from any monocot or dicot plant. Preferably, it can be obtained from a model plant, preferably *Arabidopsis thaliana*, or a crop plant selected from the group consisting of oilseed rape, evening primrose, hemp, thistle, peanut, canola, linseed, soybean, safflower, sunflower, borage, maize, wheat, rye, oats, rice, barley, cotton, cassava, pepper, solanaceae plants, preferably, potato, tobacco, eggplant or tomato, vicia species, pea, alfalfa, bushy plants (coffee, cacao, tea), salix species, trees (oil palm, coconut) and perennial grasses and fodder crops. Suitable methods for obtaining host cells from the aforementioned plants as well as conditions for culturing these cells are well known in the art.

The term "plant cell" as used herein, preferably, does not include algae cells. More preferably, the term does not include red algae and green algae cells. Also, said term, preferably, does not include cyanobacteria.

The term "enzymatic activities" refers, preferably, to enzymatic activities which are introduced into a plant cell by introducing one or more, preferably heterologous polypeptides conferring the said activities, i.e. being capable of converting glyoxylate to glycerate, preferably, via tartronate semialdehyde as an intermediate. The polypeptides referred to herein shall, therefore, be enzymes exhibiting the aforementioned enzymatic activities when present in a plant cell, preferably, when present in the chloroplasts of a plant cell. Preferred polypeptides conferring the enzymatic activities necessary for the conversion are disclosed elsewhere in this specification in detail. It is to be understood that the polypeptides referred to herein may also exhibit further biological activities.

Preferably, the plant cell comprises the said enzymatic activities for converting glyoxylate into glycerate in its chloroplasts. In order to safeguard the localization of the polypeptides conferring the aforementioned activities into the chloroplast, the polypeptide, preferably, comprises a chloroplast transit peptide. Suitable chloroplast transit peptides are known in the art and are described in Bruce 2000, Trends in Cell Biology 10, 440-447 or Kleffmann 2004, Curr. Biol. 14: 354-362.

Advantageously, it was shown in the studies underlying the present invention that plants that comprise plant cells with enzymatic activities for converting glyoxylate into glycerate showed increased cold and salt tolerance compared with plants that comprise plant cells lacking those enzymatic activities (see Examples). Specifically, in the context of the present invention, transgenic Arabidopsis thaliana plants were generated overexpressing a heterologous polypeptide having tartronate semialdehyde synthase activity and a heterologous polypeptide having tartronate semialdehyde reductase activity. The said polypeptides were linked to transit peptides allowing a transport into the chloroplasts of the plant cells. Surprisingly, the generated plants showed increased cold and salt tolerance compared with wild-type Arabidopsis plants and also compared with transgenic plants overexpressing only one of the analyzed polypeptides, i.e either a polypeptide having tartronate semialdehyde synthase activity or a polypeptide tartronate semialdehyde reductase activity. The results of the experiments done in the context of the present invention will be, if applied, very beneficial, since plants that show improved salt and/or cold tolerance will can be, e.g., grown on soil with increased salt concentrations (e.g. do to irrigation) and/or in areas with a more moderate climate.

Accordingly, in one preferred embodiment of the plant cell of the present invention, the plant cell comprises a first polypeptide having tartronate semialdehyde synthase activity, and a second polypeptide having tartronate semialdehyde reductase activity. Preferably, the plant cell comprises the said first and the said second polypeptide in its chloroplast.

A tartronate semialdehyde synthase as referred to herein as first polypeptide shall be capable of converting glyoxylate into tartronate semialdehyde and carbon dioxide. More specifically, said tartronate semialdehyde synthase shall be capable of catalyzing the chemical reaction of two glyoxylate molecules into one tartronate semialdehyde molecule and one carbon dioxide molecule. In the art, the tartronate semialdehyde synthase is also referred to as tartronate semialdehyde carboxylase, glyoxylate carbo-ligase, glyoxylic carbo-ligase, hydroxymalonic semialdehyde carboxylase, tartronic semialdehyde carboxylase, glyoxalate carboligase, and glyoxylate carboxy-lyase. Polypeptides having tartronate semialdehyde synthase activity have the EC number EC 4.1.1.47. The Enzyme Commission number (EC number) nomenclature is well known in the art. It constitutes a numerical classification for enzymes based on the chemical reactions that are catalyzed by the said enzymes.

A tartronate semialdehyde reductase as referred to herein as second polypeptide shall be capable of converting tartronate semialdehyde into glycerate. More specifically, said tartronate semialdehyde reductase shall be capable of catalyzing the reduction of tatronate semialdehyde to yield glycerate (enzymatic reaction of tartronate semialdehyde reductase: tartronate semialdehyde + NAD(P)H + H⁺ ⇔ *D*-glycerate + NAD(P)⁺). Polypeptides having tartronate semialdehyde reductase activity have the EC number EC 1.1.1.60. In the art, the tartronate semialdehyde reductase is also referred to as 2-hydroxy-3-oxopropionate reductase or *R*-glycerate:NAD(P)⁺ oxidoreductase.

Any enzyme capable of carrying out these conversions and syntheses, preferably, can be introduced into the plant cell according to the present invention, and, more preferably, into the chloroplasts of said plant cell. The aforementioned enzymatic activities can be determined by assays well known in the art.

Preferably, said first polypeptide is, however, encoded by a first polynucleotide comprising a nucleic acid selected from the group consisting of:
a) a nucleic acid having a nucleotide sequence as shown in SEQ ID No: 1;
b) a nucleic acid encoding a polypeptide having an amino acid sequence as shown in SEQ ID No: 2;
c) a nucleic acid having a nucleotide sequence being at least 50% identical to the nucleotide sequence shown in SEQ ID No: 1, wherein said nucleic acid encodes a polypeptide having tartronate semialdehyde synthase activity; and
d) a nucleic acid encoding a polypeptide having an amino acid sequence being at least 50% identical to the amino acid sequence shown in SEQ ID No:2, wherein said polypeptide has tartronate semialdehyde synthase activity.

Said second polypeptide is, preferably, encoded by a second polynucleotide comprising a nucleic acid selected from the group consisting of:
a) a nucleic acid having a nucleotide sequence as shown in SEQ ID No: 3;
b) a nucleic acid encoding a polypeptide having an amino acid sequence as shown in SEQ ID No: 4
c) a nucleic acid having a nucleotide sequence being at least 50% identical to the nucleotide sequence shown in SEQ ID No: 3 wherein said nucleic acid encodes a polypeptide having tartronate semialdehyde reductase activity; and
d) a nucleic acid encoding a polypeptide having an amino acid sequence being at least 50% identical to the amino acid sequence shown in SEQ ID No:4, wherein said polypeptide has tartronate semialdehyde reductase activity.

The term "polynucleotide" as used herein refers to a linear or circular nucleic acid molecule. It encompasses DNA as well as RNA molecules. The polynucleotide of the present invention shall be provided, preferably, either as an isolated polynucleotide (i.e. isolated from its natural context) or in genetically modified form. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides. The polynucleotide of the present invention is **characterized in that** it shall encode a polypeptide as referred to above. The polynucleotide, preferably, has a specific nucleotide sequence as mentioned above. Moreover, due to the degeneracy of the genetic code, polynucleotides are encompassed which encode a specific amino acid sequence as recited above.

Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence **characterized in that** the sequence can be derived from the aforementioned specific nucleic acid sequences by at least one nucleotide substitution, addition and/or deletion whereby the variant nucleic acid sequence shall still encode a polypeptide having the activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 × sodium chloride/sodium citrate (= SSC) at approximately 45°C, followed by one or more wash steps in 0.2 × SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 × SSC (pH 7.2). If an organic solvent is present in the abovementioned buffer, for example 50% formamide, the temperature under standard conditions is approximately 42°C. The hybridization conditions for DNA:DNA hybrids are preferably for example 0.1 × SSC and 20°C to 45°C, preferably between 30°C and 45°C. The hybridization conditions for DNA:RNA hybrids are preferably, for example, 0.1 × SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved domains of the polypeptides of the present invention. Conserved domains of the polypeptide of the present invention may be identified by a sequence comparison of the nucleic acid sequence of the polynucleotide or the amino acid sequence of the polypeptide of the present invention with sequences of other members of the enzyme families referred to in accordance with this invention. Oligonucleotides suitable as PCR primers as well as suitable PCR conditions are described in the accompanying Examples. As a template, DNA or cDNA from bacteria, fungi, plants or animals may be used. Further, variants include polynucleotides comprising nucleic acid sequences which are at least 70%, at least 75%, at least 80%, at least 85%, at least 90%, at least 95%, at least 98% or at least 99% identical to the specific nucleic acid sequences. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the specific amino acid sequences referred to herein. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS, 5 1989: 151-153) or the programs Gap and BestFit (Needleman 1970, J. Mol. Biol. 48; 443-453 and Smith 198, Adv. Appl. Math. 2: 482-489), which are part of the GCG software packet from Genetics Computer Group, 575 Science Drive, Madison, Wisconsin, USA 53711, version 1991, are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the aforementioned nucleic acid sequences is also encompassed as a polynucleotide of the present invention. The fragment shall encode a polypeptide which still has the activity as specified above. Accordingly, the polypeptide may comprise or consist of the domains of the polypeptide of the present invention conferring the said biological activity. A fragment as meant herein, preferably, comprises at least 50, at least 100, at least 250 or at least 500 consecutive nucleotides of any one of the aforementioned nucleic acid sequences or encodes an amino acid sequence comprising at least 20, at least 30, at least 50, at least 80, at least 100 or at least 150 consecutive amino acids of any one of the aforementioned amino acid sequences.

The polynucleotides of the present invention either essentially consist of the aforementioned nucleic acid sequences or comprise the aforementioned nucleic acid sequences. Thus, they may contain further nucleic acid sequences as well. Specifically, the polynucleotides of the present invention may encode fusion proteins wherein one partner of the fusion protein is a polypeptide being encoded by a nucleic acid sequence recited above. Such fusion proteins may comprise as additional part peptide sequences for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and comprise FLAG-tags, 6-histidine-tags, MYC-tags and the like.

More preferably, the first and/or the second polypeptide(s) referred to above are expressed from heterologous polynucleotides, i.e. from polynucleotides which have been either transiently, e.g., by using an expression vector, or stably, e.g., by using T- or P-DNA insertion, introduced into the plant cell. The term "heterologous" as used herein means that the polynucleotides do not occur naturally in the plant cell. The term, thus, encompasses modified or unmodified polynucleotides which are derived from different organisms or modified polynucleotides derived from the plant cell of the invention. It is to be understood that the heterologous polynucleotide shall either comprise expression control sequences which allow for expression in the plant cell or sequences which allow for integration of the heterologous polynucleotide at a locus in the genome of the plant cell where the expression of the heterologous polynucleotide will be governed by endogenous expression control sequences of the plant cell. Preferably, the heterologous polynucleotide comprises a nucleic acid having a nucleic acid sequence of the first, or the second polynucleotide as referred to above, i.e. a polynucleotide encoding a tartronate semialdehyde synthase and a polynucleotide encoding tartronate semialdehyde reductase. By introducing the aforementioned heterologous polynucleotides, transgenic plant cells are generated. Such transgenic plant cells may be obtained by transformation techniques as published, and cited, in: Plant Molecular Biology and Biotechnology (CRC Press, Boca Raton, Florida), chapter 6/7, pp.71-119 (1993); F.F. White, Vectors for Gene Transfer in Higher Plants; in: Transgenic Plants, vol. 1, Engineering and Utilization, Ed.: Kung and R. Wu, Academic Press, 1993, 15-38; B. Jenes et al., Techniques for Gene Transfer, in: Transgenic Plants, vol. 1, Engineering and Utilization, Ed.: Kung and R. Wu, Academic Press (1993), 128-143; Potrykus, Annu. Rev. Plant Physiol. Plant Molec. Biol. 42 (1991), 205-225. Preferably, transgenic plants can be obtained by T-DNA-mediated or P-DNA-mediated transformation. Such vector systems are, as a rule, **characterized in that** they contain at least the vir genes, which are required for the Agrobacterium-mediated transformation, and the sequences which delimit the T- or P-DNA (T-DNA border or P-DNA border). Suitable vectors are described elsewhere in the specification in detail.

The present invention also relates to a plant comprising the plant cell of the present invention.

The plant of the present invention, preferably, develops from the plant cell of the present invention. Thus, it is envisaged that all plant cells of the plant are plant cells according to the present invention, i.e., comprise enzymatic activities for converting glyoxylate into glycerate, preferably, in their chloroplasts. As set forth above for the plant cells of the invention, preferred plants of the present invention are selected from the group consisting of: model plants, preferably *Arabidopsis thaliana*, or crop plants, preferably, oilseed rape, evening primrose, hemp, thistle, peanut, canola, linseed, soybean, safflower, sunflower, borage, maize, wheat, rye, oats, rice, barley, cotton, cassava, pepper, solanaceae plants, preferably, potato, tobacco, eggplant or tomato, vicia species, pea, alfalfa, bushy plants (coffee, cacao, tea), salix species, trees (oil palm, coconut) and perennial grasses and fodder crops. Thus, preferably the plant is a higher plant, more preferably a dicot or monocot. Preferably, the plant of the present does not belong to the algae.

Preferably, the plant of the present invention has increased salt tolerance compared to a plant lacking a plant cell of the present invention. Thus, the plant of the present invention is capable to survive and/or to grow in the presence of increased salt concentrations in the soil or in any other growth medium that inhibit growth of a plant lacking a plant cell of the present invention and/or even kills a plant lacking a plant cell of the present invention. A plant lacking a plant cell of the present invention as meant herein, preferably, refers to an unmodified control plant of the same variety as the plant of the present invention.

An increase of salt tolerance can be determined by techniques well known in the art (Zhang and Blumwald, Nature Biotech., 2001, 19: 765-768; Laurie et al., Plant J., 2002, 32: 139-149) and described in, preferably, in the accompanying Examples below. Preferably, the increase is statistically significant. Whether an increase is statistically significant can be determined by well known statistical tests including, e.g., Student's t-test, Mann-Whitney test etc. More preferably, the salt tolerance is increased if the plant of the present invention is growing at salt concentrations and/or survives salt concentrations in a growth medium, particularly in soil, that are at least 5%, at least 10%, at least 15%, at least 20%, or at least 30% higher than the highest salt concentration at which a plant lacking a plant cell of the present invention is growing and/or which a plant lacking a plant cell of the present invention survives. More preferably, the salt tolerance is increased if the plant of the present invention is growing at salt concentrations and/or survives salt concentrations in a growth medium, particularly in soil, with a NaCl concentration of 300 mM, at which the growing or survival of a plant lacking a plant cell of the present invention is compromised. Salts in the context of the present invention, preferably, encompass Na⁺-Salts ( most preferably NaCl), K⁺-Salts, Ca²⁺-Salts, and Mg²⁺-Salts.

The plant of the present invention, preferably, has increased cold tolerance compared to a plant lacking a plant cell of the present invention. As used herein, the term "cold tolerance", preferably, refers to the capability of a plant to survive long and/or short term exposures to low temperatures. Moreover, the term preferably, also refers to the capability of a plant of growing at low temperatures. Accordingly, the plant of the present invention can grow at lower temperatures and/or survive lower temperatures than a plant lacking a plant cell of the present invention.

An increase of cold tolerance can be determined by techniques well known in the art and described in, preferably, in the accompanying Examples below. Preferably, the increase of cold tolerance is statistically significant. As set forth above, whether an increase is statistically significant can be determined by well known statistical tests including, e.g., Student's t-test, Mann-Whitney test etc. More preferably, the cold tolerance is increased if the plant of the present invention is growing at a temperature and/or survives a temperature that is at least 1°C, at least 2°C, at least 3°C, at least 4°C, or at least 5°C lower than the lowest temperature at which a plant lacking a plant cell of the present invention is growing and/or which a plant lacking a plant cell of the present invention survives. Thus, the cold tolerance, preferably, is increased if the plant of the present invention is grows at a temperature at which the growing and survival of a plant lacking a plant cell of the present invention is compromised. As said forth above, a plant lacking a plant cell of the present invention as meant herein, preferably, refers to an unmodified control plant of the same variety as the plant of the present invention.

Furthermore, since the plant of the present invention has an increased salt and/or cold tolerance of a plant lacking the plant cell of the presenting invention, the plant of the present invention has, when cultivated at low temperatures and/or high salt concentrations, an increased yield compared to a plant lacking a plant cell of the present invention. The term "yield" as used herein encompasses an increase in biomass (fresh or dry weight) of a plant part or the entire plant, and particularly, harvestable parts of the plant. The increase in biomass may be aboveground or underground. An increase in biomass underground may be due to an increase in the biomass of plant parts, such as tubers, rhizomes, bulbs etc. Particularly preferred is an increase in any one or more of the following: increased root biomass, increased root volume, increased root number, increased root diameter and increased root length. The term increased yield also encompasses an increase in seed yield. An increase in seed yield includes: (i) increased total seed yield, which includes an increase in seed biomass (seed weight) and which may be an increase in the seed weight per plant or on an individual seed basis; (ii) increased number of flowers ("florets") per panicle; (iii) increased number of filled seeds; (iv) increased seed size; (v) increased seed volume; (vi) increased individual seed area; (vii) increased individual seed length and/or width; (viii) increased harvest index, which is expressed as a ratio of the yield of harvestable parts, such as seeds, over the total biomass; (ix) increased fill rate, (which is the number of filled seeds divided by the total number of seeds and multiplied by 100); and (x) increased thousand kernel weight (TKW), which is extrapolated from the number of filled seeds counted and their total weight. An increased TKW may result from an increased seed size and/or seed weight. An increased TKW may result from an increase in embryo size and/or endosperm size.

Preferably, the increase in yield is statistically significant. More preferably, said increase is an increase of at least 10%, at least 20%, at least 30%, at least 40% or at least 50% in yield.

The present invention encompasses, furthermore, a seed obtainable from a plant of the present invention. The seed of the present invention shall be preferably capable of generating (i.e. developing into) a plant of the present invention and, specifically, a plant having the traits as defined above.

Plants or plant cells of the present invention are, preferably, transgenic plants or plant cells. Accordingly, the present invention contemplates a method for producing a transgenic plant or a plant cell having an increased salt tolerance and/or cold tolerance compared to a corresponding non-transgenic plant or plant cell, said method comprises introducing in the plant cell or plant, preferably, into the chloroplasts of the of the plant or plant cell a first polypeptide having tartronate semialdehyde synthase activity, and a second polypeptide having tartronate semialdehyde reductase activity as defined above.

The introduction of the polypeptides is, preferably, achieved by introducing heterologous polynucleotides encoding the aforementioned polypeptides as discussed elsewhere in this specification in more detail. This includes transient introduction in expression vectors or stable integration into the genome of the plant cells via, e.g., T- or P-DNA insertion. It is to be understood that one heterologous polynucleotide comprising nucleic acids encoding all of the aforementioned polypeptides may be introduced. Alternatively, separate heterologous polynucleotides each comprising a nucleic acid encoding a polypeptide of the aforementioned polypeptides may be introduced. Thus, the method, preferably, comprises the steps of:
a. introducing at least one heterologous polynucleotide encoding the said polypeptides into the plant of plant cell; and
b. expressing said polypeptides from the said at least one polynucleotide.

The present invention further encompasses a polynucleotide comprising
a nucleic acid selected from the group consisting of
a) a nucleic acid having a nucleotide sequence as shown in SEQ ID No: 1;
b) a nucleic acid encoding a polypeptide having an amino acid sequence as shown in SEQ ID No: 2;
c) a nucleic acid having a nucleotide sequence being at least 50% identical to the nucleotide sequence shown in SEQ ID No: 1, wherein said nucleic acid encodes a polypeptide having tartronate semialdehyde synthase activity; and
d) a nucleic acid encoding a polypeptide having an amino acid sequence being at least 50% identical to the amino acid sequence shown in SEQ ID No:2,
   wherein said polypeptide has tartronate semialdehyde synthase activity; and
a nucleic acid selected from the group consisting of:
a) a nucleic acid having a nucleotide sequence as shown in SEQ ID No: 3;
b) a nucleic acid encoding a polypeptide having an amino acid sequence as shown in SEQ ID No: 4;
c) a nucleic acid having a nucleotide sequence being at least 50% identical to the nucleotide sequence shown in SEQ ID No: 3 wherein said nucleic acid encodes a polypeptide having tartronate semialdehyde reductase activity; and
d) a nucleic acid encoding a polypeptide having an amino acid sequence being at least 50% identical to the amino acid sequence shown in SEQ ID No:4, wherein said polypeptide has tartronate semialdehyde reductase activity.

By introducing any one of the aforementioned polynucleotides of the present invention as a heterologous polynucleotide into a plant cell or plant, the traits referred to in accordance with the present invention will be conferred to the said plant or plant cell.

The present invention also contemplates a vector comprising one of the aforementioned polynucleotides of the present invention.

The term "vector", preferably, encompasses phage, plasmid, viral or retroviral vectors as well as artificial chromosomes, such as bacterial or yeast artificial chromosomes. Moreover, the term also relates to targeting constructs which allow for random or site-directed integration of the targeting construct into genomic DNA. Such target constructs, preferably, comprise DNA of sufficient length for either homologous or heterologous recombination as described in detail below. The vector encompassing the polynucleotides of the present invention, preferably, further comprises selectable markers for propagation and/or selection in a host. The vector may be incorporated into a host cell by various techniques well known in the art. If introduced into a host cell, the vector may reside in the cytoplasm or may be incorporated into the genome. In the latter case, it is to be understood that the vector may further comprise nucleic acid sequences which allow for homologous recombination or heterologous insertion. Vectors can be introduced into prokaryotic or eukaryotic cells via conventional transformation or transfection techniques. The terms "transformation" and "transfection", conjugation and transduction, as used in the present context, are intended to comprise a multiplicity of prior-art processes for introducing foreign nucleic acid (for example DNA) into a host cell, including calcium phosphate, rubidium chloride or calcium chloride co-precipitation, DEAE-dextran-mediated transfection, lipofection, natural competence, carbon-based clusters, chemically mediated transfer, electroporation or particle bombardment (e.g., "gene-gun"). Suitable methods for the transformation or transfection of host cells, including plant cells, can be found in Sambrook et al. (Molecular Cloning: A Laboratory Manual, 2nd ed., Cold Spring Harbor Laboratory, Cold Spring Harbor Laboratory Press, Cold Spring Harbor, NY, 1989) and other laboratory manuals, such as Methods in Molecular Biology, 1995, Vol. 44, Agrobacterium protocols, Ed.: Gartland and Davey, Humana Press, Totowa, New Jersey. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host cells.

Preferably, the vector referred to herein is suitable as a cloning vector, i.e. replicable in microbial systems. Such vectors ensure efficient cloning in bacteria and, preferably, yeasts or fungi and make possible the stable transformation of plants. Those which must be mentioned are, in particular, various binary and co-integrated vector systems which are suitable for the T-DNA-mediated or P-DNA mediated transformation. Such vector systems are, as a rule, **characterized in that** they contain at least the vir genes, which are required for the Agrobacterium-mediated transformation, and the sequences which delimit the T-DNA or P-DNA (T-DNA or P-DNA borders). These vector systems, preferably, also comprise further cis-regulatory regions such as promoters and terminators and/or selection markers with which suitable transformed host cells or organisms can be identified. While co-integrated vector systems have vir genes and T-DNA sequences arranged on the same vector, binary systems are based on at least two vectors, one of which bears vir genes, but no T-DNA, while a second one bears T-DNA, but no vir gene. As a consequence, the last-mentioned vectors are relatively small, easy to manipulate and can be replicated both in E. coli and in Agrobacterium. These binary vectors include vectors from the pBIB-HYG, pCAMBIA, pPZP, pBecks, pGreen series. Preferably used in accordance with the invention is pGreen II. An overview of binary vectors and their use can be found in Hellens 2000, Trends in Plant Science 5, 446-451. Furthermore, by using appropriate cloning vectors, the polynucleotide of the invention can be introduced into host cells or organisms such as plants or animals and, thus, be used in the transformation of plants, such as those which are published, and cited, in: Plant Molecular Biology and Biotechnology (CRC Press, Boca Raton, Florida), chapter 6/7, pp. 71-119 (1993); F.F. White, Vectors for Gene Transfer in Higher Plants; in: Transgenic Plants, vol. 1, Engineering and Utilization, Ed.: Kung and R. Wu, Academic Press, 1993, 15-38; B. Jenes et al., Techniques for Gene Transfer, in: Transgenic Plants, vol. 1, Engineering and Utilization, Ed.: Kung and R. Wu, Academic Press (1993), 128-143; Potrykus, Annu. Rev. Plant Physiol. Plant Mol. Biol. 42 (1991), 205-225.

More preferably, the vector of the present invention is an expression vector. In such an expression vector, the polynucleotide comprises an expression cassette as specified above allowing for expression in eukaryotic cells or isolated fractions thereof. An expression vector may, in addition to the polynucleotide of the invention, also comprise further regulatory elements including transcriptional as well as translational enhancers. Preferably, the expression vector is also a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

Suitable expression vector backbones are, preferably, derived from expression vectors known in the art such as Okayama-Berg cDNA expression vector pcDV1 (Pharmacia), pCDM8, pRc/CMV, pcDNA1, pcDNA3 (Invitrogene) or pSPORT1 (GIBCO BRL). Further examples of typical fusion expression vectors are pGEX (Pharmacia Biotech Inc; Smith, D.B., and Johnson, K.S. (1988) Gene 67:31-40), pMAL (New England Biolabs, Beverly, MA) and pRIT5 (Pharmacia, Piscataway, NJ), where glutathione S-transferase (GST), maltose E-binding protein and protein A, respectively, are fused with the nucleic acid of interest encoding a protein to be expressed. The target gene expression of the pTrc vector is based on the transcription from a hybrid trp-lac fusion promoter by host RNA polymerase. The target gene expression from the pET 11d vector is based on the transcription of a T7-gn10-lac fusion promoter, which is mediated by a coexpressed viral RNA polymerase (T7 gn1). This viral polymerase is provided by the host strains BL21 (DE3) or HMS174 (DE3) from a resident λ-prophage which harbors a T7 gn1 gene under the transcriptional control of the lacUV 5 promoter. Examples of vectors for expression in the yeast S. cerevisiae comprise pYeDesaturasec1 (Baldari et al. (1987) Embo J. 6:229-234), pMFa (Kurjan and Herskowitz (1982) Cell 30:933-943), pJRY88 (Schultz et al. (1987) Gene 54:113-123) and pYES2 (Invitrogen Corporation, San Diego, CA). Vectors and processes for the construction of vectors which are suitable for use in other fungi, such as the filamentous fungi, comprise those which are described in detail in: van den Hondel, C.A.M.J.J., & Punt, P.J. (1991) "Gene transfer systems and vector development for filamentous fungi, in: Applied Molecular Genetics of fungi, J.F. Peberdy et al., Ed., pp. 1-28, Cambridge University Press: Cambridge, or in: More Gene Manipulations in Fungi (J.W. Bennett & L.L. Lasure, Ed., pp. 396-428: Academic Press: San Diego). Further suitable yeast vectors are, for example, pAG-1, YEp6, YEp13 or pEMBLYe23. As an alternative, the polynucleotides of the present invention can be also expressed in insect cells using baculovirus expression vectors. Baculovirus vectors which are available for the expression of proteins in cultured insect cells (for example Sf9 cells) comprise the pAc series (Smith et al. (1983) Mol. Cell Biol. 3:2156-2165) and the pVL series (Lucklow and Summers (1989) Virology 170:31-39).

Expression vectors allowing expression in plant cells comprise those which are described in detail in: Becker, D., Kemper, E., Schell, J., and Masterson, R. (1992) "New plant binary vectors with selectable markers located proximal to the left border", Plant Mol. Biol. 20:1195-1197; and Bevan, M.W. (1984) "Binary Agrobacterium vectors for plant transformation", Nucl. Acids Res. 12:8711-8721; Vectors for Gene Transfer in Higher Plants; in: Transgenic Plants, Vol. 1, Engineering and Utilization, Ed.: Kung and R. Wu, Academic Press, 1993, p. 15-38. A plant expression cassette, preferably, comprises regulatory sequences which are capable of controlling the gene expression in plant cells and which are functionally linked so that each sequence can fulfill its function, such as promoters and polyadenylation signals. Preferred polyadenylation signals are those which are derived from Agrobacterium tumefaciens T-DNA, such as the gene 3 of the Ti plasmid pTiACH5, which is known as octopine synthase (Gielen et al., EMBO J. 3 (1984) 835 et seq.) or functional equivalents of these, but all other terminators which are functionally active in plants are also suitable. Preferred promoters are constitutive promoters (such as the 35S CaMV promoters). Also contemplated by the present invention are salt stress and/or cold stress inducible promoters. Since plant gene expression is very often not limited to transcriptional levels, a plant expression cassette preferably comprises other functionally linked sequences such as translation enhancers, for example the overdrive sequence, which comprises the 5'-untranslated tobacco mosaic virus leader sequence, which increases the protein/RNA ratio (Gallie et al., 1987, Nucl. Acids Research 15:8693-8711). Other preferred sequences for the use in functional linkage in plant gene expression cassettes are targeting sequences which are required for targeting the gene product into its relevant cell compartment. In the present case, in particular, the relevant compartments are the chloroplasts.

The present invention also relates to a composition comprising
a first polynucleotide comprising a nucleic acid selected from the group consisting of:
a) a nucleic acid having a nucleotide sequence as shown in SEQ ID No: 1;
b) a nucleic acid encoding a polypeptide having an amino acid sequence as shown in SEQ ID No: 2;
c) a nucleic acid having a nucleotide sequence being at least 50% identical to the nucleotide sequence shown in SEQ ID No: 1, wherein said nucleic acid encodes a polypeptide having tartronate semialdehyde synthase activity; and
d) a nucleic acid encoding a polypeptide having an amino acid sequence being at least 50% identical to the amino acid sequence shown in SEQ ID No:2, wherein said polypeptide has tartronate semialdehyde synthase activity; and
a second polynucleotide comprising a nucleic acid selected from the group consisting of:
a) a nucleic acid having a nucleotide sequence as shown in SEQ ID No: 3;
b) a nucleic acid encoding a polypeptide having an amino acid sequence as shown in SEQ ID No: 4;
c) a nucleic acid having a nucleotide sequence being at least 50% identical to the nucleotide sequence shown in SEQ ID No: 3 wherein said nucleic acid encodes a polypeptide having tartronate semialdehyde reductase activity; and
d) a nucleic acid encoding a polypeptide having an amino acid sequence being at least 50% identical to the amino acid sequence shown in SEQ ID No:4, wherein said polypeptide has tartronate semialdehyde reductase activity.

As set forth elsewhere in this specification, such compositions comprises heterologous polynucleotides which can be used to confer the enzymatic activities to a plant or plant cell which are required for improving the traits referred to herein.

It will be understood that the aforementioned polynucleotides, vectors or compositions of the present invention are, preferably, to be used for conferring increased salt tolerance to a plant or plant cell and/or for conferring increased cold tolerance to a plant or plant cell.

Moreover, the present invention relates to a method for identifying a plant compound, said plant compound being a candidate compound for conferring cold tolerance and/or salt tolerance to a plant and/or a plant cell comprising the steps,
a) determining the amount of a plant compound to be tested in a plant cell comprising enzymatic activities for converting glyoxylate into glycerate, or in a plant comprising said plant cell (and, thus in a plant cell or plant according to the invention),
b) determining the amount of said plant compound to be tested in a plant cell lacking enzymatic activities for converting glyoxylate into glycerate, or in a plant that does not comprise plant cells comprising enzymatic activities for converting glyoxylate into glycerate (and thus in a plant lacking a plant cell of the present invention), and
c) comparing the amount of said plant compound as determined in step a) to the amount of said candidate plant compound as determined in step b), wherein a change, preferably an increase, of the amount as determined in step a) compared with the amount as determined in step b) indicates that said plant compound is a candidate compound for conferring cold tolerance and/or salt tolerance to a plant and/or plant cell.

The aforementioned method of the present invention, preferably, is a screening method. By applying the aforementioned method plant compounds can be identified that are candidate compounds for conferring cold tolerance and/or salt tolerance to a plant and/or a plant cell.

The person skilled in the art knows that not all compounds that are identified by the aforementioned method are capable of conferring cold tolerance and/or salt tolerance to a plant and/or a plant cell. However, a compound identified by said method is a promising candidate for conferring cold tolerance and/or salt tolerance to a plant and/or a plant cell. Thus, whether a compound identified by the method of the present invention is capable or is not capable of conferring cold tolerance and/or salt tolerance to a plant and/or a plant cell, preferably, is to be confirmed in an additional step. Accordingly, in order to confirm whether a plant compound identified by the method of the present invention is capable of conferring cold tolerance and/or salt tolerance to a plant and/or a plant cell, the additional step of confirming whether said identified plant compound is capable of conferring cold tolerance and/or salt tolerance to a plant and/or a plant cell, preferably, is carried out. How to confirm whether an identified plant compound is capable of conferring cold tolerance and/or salt tolerance to a plant and/or a plant cell is known the art. Preferably, for confirming that a compound is capable of conferring cold tolerance and/or salt tolerance to a plant and/or a plant cell, a plant compound identified by the method of the present invention is contacted with a plant and/or a plant cell. Preferably, as a control, a plant and/or a plant cell is not contacted with said identified plant compound (or with a lower amount). Preferably, a plant compound is capable of conferring cold tolerance and/or salt tolerance to a plant and/or a plant cell if the plant cell and/or plant that is contacted with said compound has an increased cold tolerance and/or increased salt tolerance compared with a plant or plant cell that has not been contacted with the said compound. The terms "increased salt tolerance" and "increased cold tolerance" as well as how to determine whether salt tolerance and/or cold tolerance is/are increased are described elsewhere in this specification.

Contacting a plant and/or a plant cell with a compound, preferably, may be done by any method deemed appropriate that allows a delivery of a candidate compound to be tested to said plant or plant cell. It is to be understood that delivery can depend on the compound to be tested. E.g., if the candidate compound is a polynucleotide, peptide or polypeptide, the contacting can be done by stably or transiently expressing said polynucleotide, peptide or polypeptide. E.g., if the candidate compound is a metabolite, the contacting can done by directly administering said compound to the plant and/or plant cell, e.g. by adding said compound to the growth medium and/or by irrigating the plant with water comprising the said compound. Also contemplated is the expression of suitable polypeptides that allow increased formation of the metabolite to be tested.

A plant compound to be tested can be any compound that is present in the plant cell according to the invention or in a plant comprising said plant cell. Preferably, said plant compound is a polynucleotide, more preferably a peptide or polypeptide, and even more preferably a metabolite. Metabolites are small molecule compounds involved a metabolic pathway that occurs in said plant and/or said plant cell. Accordingly, the term "metabolite", preferably, includes substrates, intermediates as well as products of a metabolic pathway in said plant and/or said plant cell. Metabolic pathways are well known in the art and may vary between various plant species (see e.g. Mueller et al. Plant Physiol., 2003, 132 (2): 453; Kopka et al., Genome Biology, 2004, 5: 109).Thus, the term "metabolites" preferably includes the following classes of compounds: carbohydrates, saccharides, alcohols, fatty acids, ketones, carboxylic acids, amino acids, peptides, nucleotides, nucleosides, purines, or pyrimidines, or derivatives of the aforementioned compounds. The metabolites may be primary metabolites which are required for normal function, organ function or plant growth, or development. Moreover, metabolites further comprise secondary metabolites having essential ecological function.

The term "amount" as used herein encompasses the absolute amount of a compound, the relative amount or concentration of the said compound as well as any value or parameter which correlates thereto or can be derived therefrom. Such values or parameters comprise intensity signal values from all specific physical or chemical properties obtained from the said compounds by direct measurements, e.g., intensity values in mass spectra or NMR spectra. Moreover, encompassed are all values or parameters which are obtained by indirect measurements specified elsewhere in this description, e.g., response levels determined from biological read out systems in response to the compound or intensity signals obtained from specifically bound ligands. It is to be understood that values correlating to the aforementioned amounts or parameters can also be obtained by all standard mathematical operations.

Determining of the amount of a plant compound can be done by any method deemed appropriate. It is to be understood that for determining the amount of a compound to be tested, the plant cell or plant may be further processed by method known in the art (e.g. protein extraction). Determining the amount of a compound referred to in this specification relates to measuring the amount or concentration, preferably semi-quantitatively or quantitatively. Measuring can be done directly or indirectly. Direct measuring relates to measuring the amount or concentration of the compound based on a signal which is obtained from the compound itself and the intensity of which directly correlates with the number of molecules of the peptide present in the sample. Such a signal - sometimes referred to herein as intensity signal -may be obtained, e.g., by measuring an intensity value of a specific physical or chemical property of the compound. Indirect measuring includes measuring of a signal obtained from a secondary component (i.e. a component not being the compound itself) or a biological read out system, e.g., measurable cellular responses, ligands, labels, or enzymatic reaction products.

Preferably, determining the amount of a compound to be tested can be achieved by all known means for determining the amount of said compound. It is to be understood that the determination can depend on the plant compound to be tested. Thus, the amount of a compound may also be determined in a sample obtained from a plant or plant cell. Preferably, if said compound is a polynucleotide the amount of said polynucleotide is determined by assessing the expression level of said polynucleotide by methods well known in the art (e.g. by real-time PCR analysis or by performing hybridization reactions such as northern blot analysis or microarray analysis). Preferably, if the compound to be tested is peptide or polypeptide, the amount of said compound is determined by applying immunoassay methods which may utilize labeled molecules in various sandwich, competition, or other assay formats. Such assays, e.g., will develop a signal which is indicative for the presence or absence of the peptide or polypeptide. Also, the amount may be determined by electrophoretic approaches. Preferably, if the compound is a metabolite, the amount of said compound is determined in a metabolic profiling analysis. The term "metabolic profiling" is well known in the art (see e.g. Roessner et al., Plant Cell, 2001, 13, 11-29; Kopka et al., Genome Biology, 2004, 5: 109). Metabolic profiling combines gas or liquid chromatographic separation of compounds with a subsequent mass spectrometric identification.

The term "comparing" as used herein encompasses comparing the amount of a compound comprised by a plant cell comprising enzymatic activities for converting glyoxylate into glycerate or a plant comprising said plant cell with an amount of said compound comprised by a plant cell lacking enzymatic activities for converting glyoxylate into glycerate or a plant not comprising plant cells comprising enzymatic activities for converting glyoxylate into glycerate. It is to be understood that comparing as used herein refers to a comparison of corresponding parameters or values, e.g., an absolute amount is compared to an absolute amount while a concentration is compared to a concentration or an intensity signal in a sample obtained from a first plant cell or plant is compared to the same type of intensity signal of a sample second plant cell or plant.

A change of the amount of a plant compound in plant cell comprising enzymatic activities for converting glyoxylate into glycerate (or in a plant comprising said plant cell) compared with an amount of said plant compound in a plant cell lacking enzymatic activities for converting glyoxylate into glycerate (or in a plant not comprising a plant cell comprising enzymatic activities for converting glyoxylate into glycerate), preferably, indicates that said plant compound is a candidate compound for conferring cold tolerance and/or salt tolerance to a plant and/or a plant cell. Preferably, the change is a statistically significant change. More preferably, the change is a more than 1.25 fold, more than 1.5 fold, 2.0 fold, 3.0 fold, 4.0 fold, 5.0 fold, 7.0 fold, 10.0 fold, or more than 20.0 fold change. More preferably, the change is an increase, even more preferably a statistically significant increase. Accordingly, said change is a more than 1.25 fold, more than 1.5 fold, 2.0 fold, 3.0 fold, 4.0 fold, 5.0 fold, 7.0 fold, 10.0 fold, or more than 20.0 fold increase of the amount of a plant compound in a plant cell comprising enzymatic activities for converting glyoxylate into glycerate compared with an amount of said plant compound in a plant cell lacking enzymatic activities for converting glyoxylate into glycerate, or of the amount of a plant compound in a plant comprising a plant cell of the present invention compared with the amount in a plant lacking a plant cell of the present invention.

It was shown in the studies underlying the present invention that lysates of plant cells comprising enzymatic activities for converting glyoxylate into glycerate (and thus lysates of plant cells according to the present invention) have a lower freezing point than lysates of plant cells lacking the said activities. Specifically, it was observed that lysates of plant cells expressing an heterogenous tartronate semialdehyde synthase and an heterogenous tartronate semialdehyde reductase polypeptide -when taken out of a freezer- thaw significantly earlier than lysates of plant cells not expressing the said heterogenous reductase and heterogenous synthase polypetides. The results indicate that, as a result of the presence of enzymatic activities for converting glyoxylate into glycerate increased amounts of a compound (compounds) are generated by a plant cell that confer(s) cold tolerance and/or salt tolerance to a plant comprising said plant cell.

All references cited in this specification are herewith incorporated by reference with respect to their entire disclosure content and the disclosure content specifically mentioned in this specification.

The figures show:

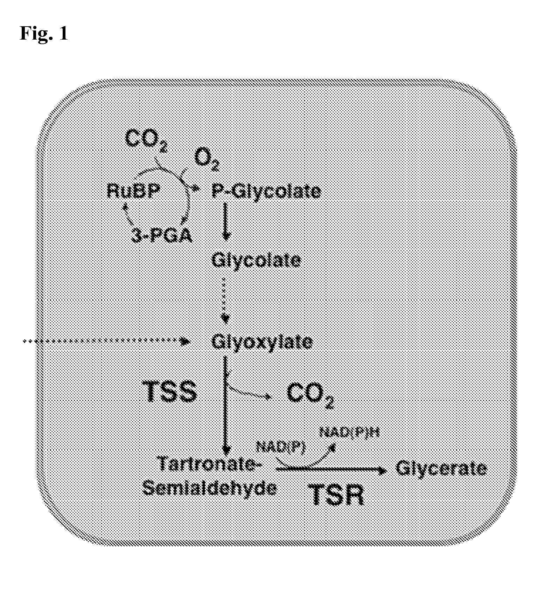

**Figure 1****:** Conversion of glyoxylate into glycerate via the intermediate tartronate-semialdehyde: Two molecules of glyoxylate are converted into tartronate semialdehyde (2-hydroxy-3-oxopropionate) and CO₂ by the tartronate-semialdehyde-synthase (TSS; EC 4.1.1.47). The tartronate semialdehyde is the substrate of the 2-hydroxy-3-oxo-propionate-reductase (tartronate-semialdehyde-reductase, TSR; EC 1.1.1.60), which produces D-glycerate.

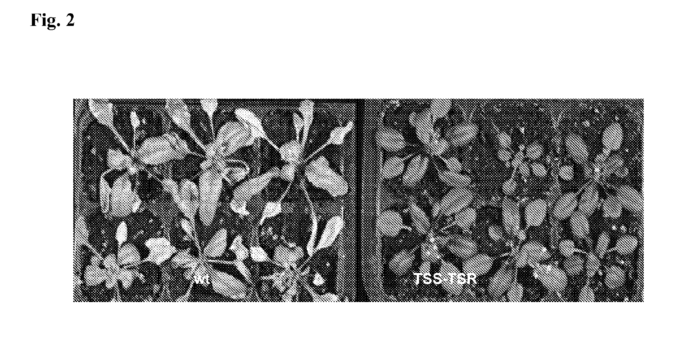

**Figure 2****:** Comparison of wild type plants (Col-0) and TSS-TSR plants seven days after irrigation with a salt solution. The plants were grown for four days under green house conditions (long day). TSS: tartronate semialdehyde synthase, TSR: tartronate semialdehyde reductase.
**Figure 3****:** Determination of germination efficiency. Three days old wild type (Col-0) and TSS-TSR double transgenic plants on MS-Argar supplemented with 100 mM NaCl.
**Figure 4****:** Proportion of germinated wild type seeds (Col-0), TSR single transgenic seeds, TSS single transgenic seeds and TSS-TSR double transgenic seeds, 3-7 days after sowing on MS-Agar medium supplemented with 100 or 150 mM NaCl.

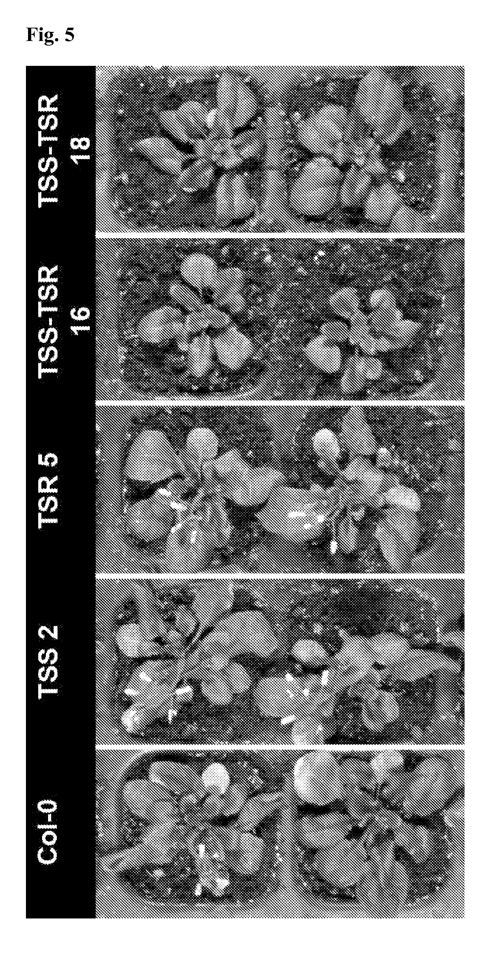

**Figure 5****:** Wild type plants (Col-0), TSS single transgenic plants, TSR single transgenic plants and TSS-TSR double transgenic plants grown for 18 days in a cold chamber at 4°C). Plants were grown under green house conditions for four weeks before they were transferred into the cold chamber (see Example 3).

The following examples merely illustrate the invention. They should, whatsoever, not be construed as limiting the scope of protection.

### Example 1: Generation of TSR (tartronate semialdehyde reductase) and TSS (tartronate semialdehyde synthase) single transformants and TSR-TSS double transformants

The genes corresponding to the bacterial enzymes TSS (NP_415040.1) and TSR (NP_417594.1) were amplified by PCR using E. coli genomic DNA as a template and cloned into the vector pGEM-T Easy. In order to target these proteins to the chloroplast, stromal targeting presequences were inserted upstream the genomic sequences. To direct the expression in *A. thaliana*, these DNA sequences were cloned into the binary vector pGreenII using the multiple cloning sites between the CaMV 35S promoter and the Octopin-Synthetase-Terminator from the pBinAR vector.

TSS was cloned into the pGreenII 35S-nos-Hyg vector and TSR was cloned into the pGreenII 35S-nos-Bar vector. The constructs were transformed into Arabidopsis thaliana, ecotype Columbia, via Agrobacterium-mediated transformation. A TSR line (TSS5) was selected and used for the transformation with the vector for the expression of TSS. Single transformants for TSR and TSS and double transformants TSR-TSS were obtained and characterized.

### Example 2: The expression of TSS and TSR in Arabidopsis thaliana increases salt tolerance

During the analysis of plants expressing the TSS polypeptide and the TSR polypeptide, liquid extracts were prepared of those plants and stored at -20°C. Interestingly, when thawing those extracts after taking them out of the freezer, extracts derived from TSS-TSR double transformant plants thawed significantly earlier than extracts of wild type plants and TSR and TSS single transformants. Also, freezing of extracts of TSR-TSS double transformants was significantly delayed compared with extracts of wild-type or plants that express only the TSS or the TSR polypeptide. This observation indicates that TSR-TSS plants accumulate compounds that confer cold and/or salt stress resistance.

For further analysis, TSS-TSR double transformant plants and the corresponding control plants (expressing TSS or TSR alone or wild type plants) were grown in the green house and watered with 300mM NaCl solution. After seven days of irrigation with 300mM NaCl solution, significant damages were observed in the wild-type (Col-0) and TSR plants. For TSS single transformants starting lesions were observed. We did not observe any damages or lesions in TSS-TSR double transformants. The double transgenic plants only showed reduced growth as a consequence of the salt stress.

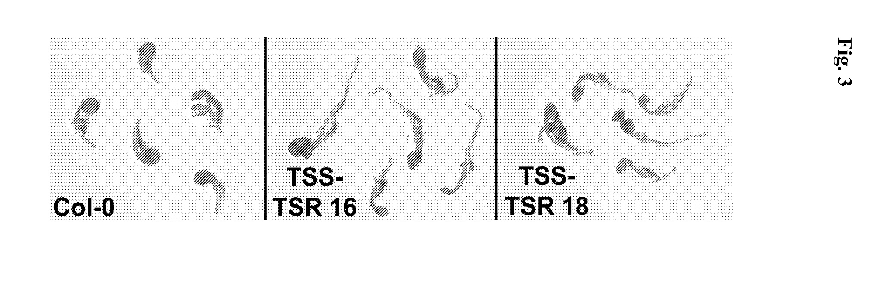

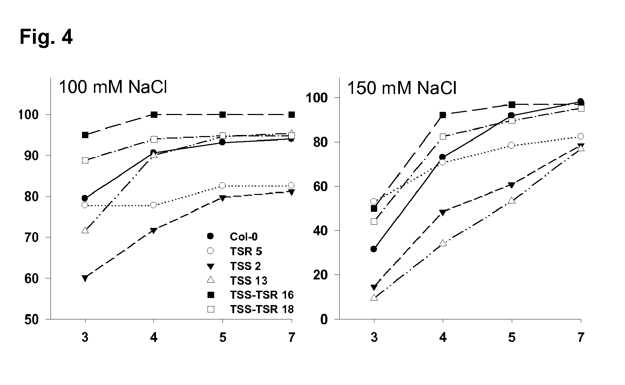

The test for salt tolerance was carried out with adult plants. After this test, the germination efficiency of TSS-TSR double transformant seeds in the presence of high salt concentrations in the substrate was determined. Therefore, seeds of Col-0 (ecotype Columbia 0) wild-type plants, seeds of TSS single transgenic plants, seeds of TSR single transgenic plants, and TSS-TSR double homozygous transgenic plants were sown on MS-Agar plates supplemented with 100 and 150 mM NaCl. After a few days germination efficiency was determined. The results are shown in figure 3 and figure 4.

The results indicate that the germination efficiency is higher for seeds of TSS-TSR double transformants than for the single transformants and wild-type plants. For example, three days after sowing, a higher proportion of seeds from TSS-TSR double transformants were germinated than seeds of control plants (at both analyzed salt concentrations, 100 mM as well as of 150 mM NaCl).

These observations further confirm that TSS-TSR double transformants have increased salt tolerance compared with TSS and TSR single transformants and wild-type plants.

### Example 3: The expression of TSS and TSR in Arabidopsis thaliana increases cold tolerance

Moreover, it was analyzed whether TSS-TSR plants have increased cold tolerance. Four weeks old wild type plants (Col-0), TSS single transformants, TSR single transformants and TSS-TSR double transformants were grown under green house conditions, and thereafter transferred into the cold chamber and incubated at 4°C and 50 µE. After 18 days in the cold chamber, leaves of wild type plans, TSS single transformants (line 2) and TSR double transformants (line 5) showed lesions, whereas leaves of TSS-TSR double transgenic plants did not show any lesions and were still green. Moreover, wild-type, TSS plants and TSR plants had started flowering whereas TSS-TSR double transgenic plants did not start flowering. The effect of the low temperatures on the phenotype that were observed for wild-type, TSS single transgenic and TSR single transgenic plants are generally considered as a stress response to cold.

## Claims

1. A plant cell comprising enzymatic activities for converting glyoxylate into glycerate.

2. The plant cell of claim 1, wherein the plant cell comprises said enzymatic activities in its chloroplasts.

3. The plant cell of claim 1 or 2, wherein glyoxylate is converted into glycerate via tartronate semialdehyde.

4. The plant cell of any one of claim 1 to 3, wherein said plant cell comprises a first polypeptide having tartronate semialdehyde synthase activity, and a second polypeptide having tartronate semialdehyde reductase activity.

5. The plant cell of claim 4, wherein said wherein said first polypeptide is encoded by a first polynucleotide comprising a nucleic acid selected from the group consisting of:
a) a nucleic acid having a nucleotide sequence as shown in SEQ ID No: 1;
b) a nucleic acid encoding a polypeptide having an amino acid sequence as shown in SEQ ID No: 2;
c) a nucleic acid having a nucleotide sequence being at least 50% identical to the nucleotide sequence shown in SEQ ID No: 1 wherein said nucleic acid encodes a polypeptide having tartronate semialdehyde synthase activity; and
d) a nucleic acid encoding a polypeptide having an amino acid sequence being at least 50% identical to the amino acid sequence shown in SEQ ID No:2, wherein said polypeptide has tartronate semialdehyde synthase activity.

6. The plant cell of claim 4 or 5, wherein said wherein said second polypeptide is encoded by a first polynucleotide comprising a nucleic acid selected from the group consisting of:
a) a nucleic acid having a nucleotide sequence as shown in SEQ ID No: 3;
b) a nucleic acid encoding a polypeptide having an amino acid sequence as shown in SEQ ID No: 4;
c) a nucleic acid having a nucleotide sequence being at least 50% identical to the nucleotide sequence shown in SEQ ID No: 3 wherein said nucleic acid encodes a polypeptide having tartronate semialdehyde reductase activity; and
d) a nucleic acid encoding a polypeptide having an amino acid sequence being at least 50% identical to the amino acid sequence shown in SEQ ID No:4, wherein said polypeptide has tartronate semialdehyde reductase activity.

7. The plant cell any one of claims 3 to 6, wherein said first polynucleotide is expressed from a heterologous polynucleotide and wherein said heterologous polynucleotide comprises a nucleic acid as defined in claim 5.

8. The plant cell of any one of claims 3 to 7, wherein said second polynucleotide is expressed from a heterologous polynucleotide and wherein said heterologous polynucleotide comprises a nucleic acid as defined in claim 6.

9. The plant cell of any one of claims 1 to 8, wherein said first and/or second polynucleotide comprises a chloroplast transit peptide.

10. The plant cell of any one of claims 1 to 9, wherein said plant cell has an increased salt tolerance and/or cold tolerance compared to a plant cell lacking enzymatic activities for converting glyoxylate into glycerate.

11. A plant comprising the plant cell of any one of claims 1 to 10.

12. The plant of claim 11, wherein said plant has an increased salt tolerance compared to a plant lacking enzymatic activities for converting glyoxylate into glycerate.

13. A seed obtainable from the plant of claims 11 or 12.

14. A method for producing a transgenic plant or a transgenic plant cell having increased salt tolerance and/or cold tolerance compared to a corresponding non-transgenic plant or plant cell, comprising introducing into the cells of a plant, or into a plant cell a first polypeptide and a second polypeptide as defined in claims 5 to 9.

15. A method for identifying a plant compound, said plant compound being a candidate compound for conferring cold tolerance and/or salt tolerance to a plant and/or a plant cell comprising the steps,
a) determining the amount of a plant compound to be tested in the plant cell of any one of claims 1 to 10 or in plant comprising said plant cell, or in the plant of claims 11 and 12,
b) determining the amount of said plant compound to be tested in a plant cell lacking enzymatic activities for converting glyoxylate into glycerate, or in a plant that does not comprise plant cells comprising enzymatic activities for converting glyoxylate into glycerate, and
c) comparing the amount of said plant compound as determined in step a) to the amount of said plant compound as determined in step b), wherein an increase of the amount as determined in step a) compared with the amount as determined in step b) indicates that said compound is a candidate compound for conferring cold tolerance and/or salt tolerance to a plant and/or plant cell.


SEQUENCE LISTING

<110> Universität zu Köln

<120> A plant cell comprising enzymatic activities for converting
      glyoxylate to glycerate

<130> P65594EP

<160> 4

<170> PatentIn version 3.4

<210> 1
<211> 1782
<212> DNA
<213> Escherichia coli

<400> 1
atggcaaaaa tgagagccgt tgacgcggca atgtatgtgc tggagaaaga aggtatcact      60

accgccttcg gtgttccggg agctgcaatc aatccgttct actcagcgat gcgtaagcac     120

ggcggtattc gtcacattct ggcgcgtcat gtggaaggtg cttcgcacat ggcggaaggt     180

tatacccgcg caacggcagg gaatatcggc gtatgtctgg ggacttccgg tcctgcgggc     240

acggacatga tcaccgcgct ctattccgct tctgctgatt ccattcctat tctgtgcatt     300

accggccagg caccgcgcgc ccgtctgcat aaagaagatt ttcaggccgt agatattgaa     360

gcaattgcta aaccggtcag caaaatggcg gttacagttc gtgaagcggc gctggtgcct     420

cgcgtgctgc aacaggcatt tcacctgatg cgttctggtc gtccgggtcc ggtactggtg     480

gatttaccgt tcgacgttca ggttgcggaa atcgagtttg atcctgacat gtacgaaccg     540

ctgccggtct acaaacctgc tgccagccgt atgcagatcg aaaaagctgt agaaatgtta     600

atccaggccg aacgtccggt gattgttgcc gggggcgggg taattaatgc tgacgcagct     660

gcactgttac aacagtttgc tgaactgacc agcgttccgg tgatcccaac gctaatgggc     720

tggggctgta tcccggacga tcatgaactg atggccggga tggtgggtct gcaaaccgcg     780

catcgttacg gtaacgcaac gctgctggcg tctgacatgg tgtttggtat cggtaaccgt     840

tttgctaacc gtcataccgg ctcggtagag aaatacaccg aagggcgcaa aatcgttcat     900

attgatattg agccgacgca aattggtcgc gtgctgtgtc cggatctcgg tattgtctct     960

gatgctaaag cggcgctgac actgctggtt gaagtggcgc aggagatgca aaaagcgggt    1020

cgtctgccgt gtcgtaaaga atgggtcgcc gactgccagc agcgtaaacg cactttgctg    1080

cgcaaaaccc acttcgacaa cgtgccggtg aaaccgcagc gcgtgtatga agagatgaac    1140

aaagcctttg gtcgcgatgt ttgttatgtc accaccattg gtctgtcaca aatcgctgcg    1200

gcacaaatgc tgcatgtctt taaagaccgc cactggatca actgtggtca ggctggtccg    1260

ttaggctgga cgattccggc tgcgctaggg gtttgtgccg ctgatccgaa acgcaatgtg    1320

gtggcgattt ctggcgactt tgacttccag ttcctgattg aagagttagc tgttggcgcg    1380

cagttcaaca ttccgtacat ccatgtgctg gtcaacaacg cttatctggg gctgattcgt    1440

cagtcacaac gcgcttttga catggactac tgcgtgcaac tcgctttcga gaatatcaac    1500

tccagtgaag tgaatggcta cggtgttgac cacgtaaaag tagcggaagg tttaggttgt 1560

aaagctattc gggtcttcaa accggaagat attgcgccag cctttgaaca ggcgaaagcc 1620

ttaatggcgc aatatcgggt accggtagtc gtggaagtta ttctcgagcg tgtgaccaat 1680

atttcgatgg gcagcgaact ggataacgtc atggaatttg aagatatcgc cgataacgca 1740

gcggacgcac cgactgaaac ctgcttcatg cactatgaat aa 1782

<210> 2
<211> 593
<212> PRT
<213> Escherichia coli

<400> 2

Met Ala Lys Met Arg Ala Val Asp Ala Ala Met Tyr Val Leu Glu Lys
1               5                   10                  15

Glu Gly Ile Thr Thr Ala Phe Gly Val Pro Gly Ala Ala Ile Asn Pro
            20                  25                  30

Phe Tyr Ser Ala Met Arg Lys His Gly Gly Ile Arg His Ile Leu Ala
        35                  40                  45

Arg His Val Glu Gly Ala Ser His Met Ala Glu Gly Tyr Thr Arg Ala
    50                  55                  60

Thr Ala Gly Asn Ile Gly Val Cys Leu Gly Thr Ser Gly Pro Ala Gly
65                  70                  75                  80

Thr Asp Met Ile Thr Ala Leu Tyr Ser Ala Ser Ala Asp Ser Ile Pro
                85                  90                  95

Ile Leu Cys Ile Thr Gly Gln Ala Pro Arg Ala Arg Leu His Lys Glu
            100                 105                 110

Asp Phe Gln Ala Val Asp Ile Glu Ala Ile Ala Lys Pro Val Ser Lys
        115                 120                 125

Met Ala Val Thr Val Arg Glu Ala Ala Leu Val Pro Arg Val Leu Gln
    130                 135                 140

Gln Ala Phe His Leu Met Arg Ser Gly Arg Pro Gly Pro Val Leu Val
145                 150                 155                 160

Asp Leu Pro Phe Asp Val Gln Val Ala Glu Ile Glu Phe Asp Pro Asp
                165                 170                 175

Met Tyr Glu Pro Leu Pro Val Tyr Lys Pro Ala Ala Ser Arg Met Gln
            180                 185                 190

Ile Glu Lys Ala Val Glu Met Leu Ile Gln Ala Glu Arg Pro Val Ile
        195                 200                 205

Val Ala Gly Gly Gly Val Ile Asn Ala Asp Ala Ala Ala Leu Leu Gln
    210                 215                 220

Gln Phe Ala Glu Leu Thr Ser Val Pro Val Ile Pro Thr Leu Met Gly
225                 230                 235                 240

Trp Gly Cys Ile Pro Asp Asp His Glu Leu Met Ala Gly Met Val Gly
                245                 250                 255

Leu Gln Thr Ala His Arg Tyr Gly Asn Ala Thr Leu Leu Ala Ser Asp
            260                 265                 270

Met Val Phe Gly Ile Gly Asn Arg Phe Ala Asn Arg His Thr Gly Ser
        275                 280                 285

Val Glu Lys Tyr Thr Glu Gly Arg Lys Ile Val His Ile Asp Ile Glu
    290                 295                 300

Pro Thr Gln Ile Gly Arg Val Leu Cys Pro Asp Leu Gly Ile Val Ser
305                 310                 315                 320

Asp Ala Lys Ala Ala Leu Thr Leu Leu Val Glu Val Ala Gln Glu Met
                325                 330                 335

Gln Lys Ala Gly Arg Leu Pro Cys Arg Lys Glu Trp Val Ala Asp Cys
            340                 345                 350

Gln Gln Arg Lys Arg Thr Leu Leu Arg Lys Thr His Phe Asp Asn Val
        355                 360                 365

Pro Val Lys Pro Gln Arg Val Tyr Glu Glu Met Asn Lys Ala Phe Gly
    370                 375                 380

Arg Asp Val Cys Tyr Val Thr Thr Ile Gly Leu Ser Gln Ile Ala Ala
385                 390                 395                 400

Ala Gln Met Leu His Val Phe Lys Asp Arg His Trp Ile Asn Cys Gly
                405                 410                 415

Gln Ala Gly Pro Leu Gly Trp Thr Ile Pro Ala Ala Leu Gly Val Cys
            420                 425                 430

Ala Ala Asp Pro Lys Arg Asn Val Val Ala Ile Ser Gly Asp Phe Asp
        435                 440                 445

Phe Gln Phe Leu Ile Glu Glu Leu Ala Val Gly Ala Gln Phe Asn Ile
    450                 455                 460

Pro Tyr Ile His Val Leu Val Asn Asn Ala Tyr Leu Gly Leu Ile Arg
465                 470                 475                 480

Gln Ser Gln Arg Ala Phe Asp Met Asp Tyr Cys Val Gln Leu Ala Phe
                485                 490                 495

Glu Asn Ile Asn Ser Ser Glu Val Asn Gly Tyr Gly Val Asp His Val
            500                 505                 510

Lys Val Ala Glu Gly Leu Gly Cys Lys Ala Ile Arg Val Phe Lys Pro
        515                 520                 525

Glu Asp Ile Ala Pro Ala Phe Glu Gln Ala Lys Ala Leu Met Ala Gln
    530                 535                 540

Tyr Arg Val Pro Val Val Val Glu Val Ile Leu Glu Arg Val Thr Asn
545                 550                 555                 560

Ile Ser Met Gly Ser Glu Leu Asp Asn Val Met Glu Phe Glu Asp Ile
                565                 570                 575

Ala Asp Asn Ala Ala Asp Ala Pro Thr Glu Thr Cys Phe Met His Tyr
            580                 585                 590

Glu

<210>  3
<211>  900
<212>  DNA
<213>  Escherichia coli

<400>  3
atgattgata tgactatgaa agttggtttt attggcctgg ggattatggg taaaccaatg      60

agtaaaaacc ttctgaaagc aggttactcg ctggtggttg ctgaccgtaa cccagaagct     120

attgctgacg tgattgctgc aggtgcagaa acagcgtcta cggctaaagc gatcgctgaa     180

cagtgcgacg tcatcataac catgctgcca aactcccctc atgtgaaaga ggtggcgctg     240

ggtgagaatg gcattattga aggcgcgaag ccaggtacgg tattgatcga tatgagttct     300

atcgcaccgc tggcaagccg tgaaatcagc gaagcgctga aagcgaaagg cattgatatg     360

ctggatgctc cggtgagcgg cggtgaaccg aaagccatcg acggtacgct gtcagtgatg     420

gtgggcggcg acaaggctat tttcgacaaa tactatgatt tgatgaaagc gatggcgggt     480

tccgtggtgc ataccgggga aatcggtgca ggtaacgtca ccaaactggc aaatcaggtc     540

attgtggcgc tgaatattgc cgcgatgtca gaagcgttaa cgctggcaac taaagcgggc     600

gttaacccgg acctggttta tcaggcaatt cgcggtggac tggcgggcag taccgtgctg     660

gatgccaaag cgccgatggt gatggaccgc aacttcaagc cgggcttccg tattgatctg     720

catattaagg atctggcgaa tgcgctggat acttctcacg gcgtcggcgc acaactgccg     780

ctcacagctg cggttatgga gatgatgcag gcactgcgag cagatggttt aggaacggcg     840

gatcatagcg ccctggcgtg ctactacgaa aaactggcga aagtcgaagt tactcgttaa     900

<210>  4
<211>  299
<212>  PRT
<213>  Escherichia coli

<400>  4

Met Ile Asp Met Thr Met Lys Val Gly Phe Ile Gly Leu Gly Ile Met
1               5                   10                  15

Gly Lys Pro Met Ser Lys Asn Leu Leu Lys Ala Gly Tyr Ser Leu Val
            20                  25                  30

Val Ala Asp Arg Asn Pro Glu Ala Ile Ala Asp Val Ile Ala Ala Gly
        35                  40                  45

Ala Glu Thr Ala Ser Thr Ala Lys Ala Ile Ala Glu Gln Cys Asp Val
    50                  55                  60

Ile Ile Thr Met Leu Pro Asn Ser Pro His Val Lys Glu Val Ala Leu
65                  70                  75                  80

Gly Glu Asn Gly Ile Ile Glu Gly Ala Lys Pro Gly Thr Val Leu Ile
                85                  90                  95

Asp Met Ser Ser Ile Ala Pro Leu Ala Ser Arg Glu Ile Ser Glu Ala
            100                 105                 110

Leu Lys Ala Lys Gly Ile Asp Met Leu Asp Ala Pro Val Ser Gly Gly
        115                 120                 125

Glu Pro Lys Ala Ile Asp Gly Thr Leu Ser Val Met Val Gly Gly Asp
    130                 135                 140

Lys Ala Ile Phe Asp Lys Tyr Tyr Asp Leu Met Lys Ala Met Ala Gly
145                 150                 155                 160

Ser Val Val His Thr Gly Glu Ile Gly Ala Gly Asn Val Thr Lys Leu
                165                 170                 175

Ala Asn Gln Val Ile Val Ala Leu Asn Ile Ala Ala Met Ser Glu Ala
            180                 185                 190

Leu Thr Leu Ala Thr Lys Ala Gly Val Asn Pro Asp Leu Val Tyr Gln
        195                 200                 205

Ala Ile Arg Gly Gly Leu Ala Gly Ser Thr Val Leu Asp Ala Lys Ala
    210                 215                 220

Pro Met Val Met Asp Arg Asn Phe Lys Pro Gly Phe Arg Ile Asp Leu
225                 230                 235                 240

His Ile Lys Asp Leu Ala Asn Ala Leu Asp Thr Ser His Gly Val Gly
                245                 250                 255

Ala Gln Leu Pro Leu Thr Ala Ala Val Met Glu Met Met Gln Ala Leu
            260                 265                 270

Arg Ala Asp Gly Leu Gly Thr Ala Asp His Ser Ala Leu Ala Cys Tyr
        275                 280                 285

Tyr Glu Lys Leu Ala Lys Val Glu Val Thr Arg
    290                 295